# EUROPEAN PATENT APPLICATION

(11) **EP 4 455 116 A1**
(43) Date of publication of application: **30.10.2024**
(21) Application number: 23170073.3
(22) Date of filing: 26.04.2023
(51) Int. Cl.: C07C 17/35, C07C 209/74

(54) **PROCESS FOR THE PREPARATION OF HALODIFLUOROBENZENE**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention relates to a novel process for preparing 1-halo-3,5-difluorobenzene of the formula (I).

## Description

The present invention relates to a novel process for preparing 1-halo-3,5-difluorobenzene of the formula **(I)**.

The synthesis of 1-bromo-3,5-difluorobenzene of the formula **(I)** has already been described in US patent 5,977,412 starting from 2-bromo-4,6-difluoroaniline. A recent publication for the synthesis of 1-bromo-3,5-difluorobenzene starting from 2,4-difluoroaniline is CN105949067.

1-Halo-3,5-difluorobenzene of the formula **(I)** is an important precursor for the preparation of agrochemical compounds, e.g., for the chemical class of isooxazoline carboxamides which are very potent herbicides (e.g.: WO2018/228985, WO2018/228986).

In order to prepare 1-bromo-3,5-difluorobenzene according to the procedure disclosed in US patent 5,977,412 2-bromo-4,6-difluoroaniline has to be synthesized in high dilution in a highly acidic aqueous medium employing bromine as reagent, thus, producing additional stoichiometric amounts of hydrobromic acid. An isolation of the desired material is not possible under these conditions since the neutralization of the reaction medium requirs large amounts of base. In addition, a direct processing of the aqueous product solution within the next step results in decreased yields. In CN105949067 the reaction is performed in aqueous medium, too. The disadvantage is that more than stoichiometric amounts of the reagents are need and a pollution of the water is the consequence.

An object of the present invention is providing a cost-effective process for preparing 1-halo-3,5-difluorobenzene that can be used on industrial scale without the above-mentioned disadvantages.

The object is achieved by a process for preparing 1-halo-3,5-difluorobenzene of the formula **(I)** 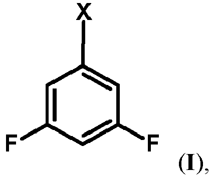 characterized in that compounds of the general formula **(II)** 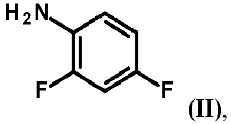 react in a first step in the presence of bromine or chlorine or a combination of a bromine or chlorine source and an oxidizing agent
to compounds of the general formula **(III)** 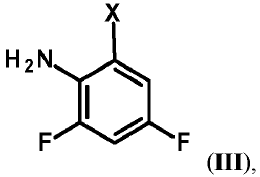 wherein
X is bromo or chloro,
and in a second step, is further converted to compounds of the formula **(I)** in the presence of a nitrite source, a copper catalyst and a secondary alcohol that can be oxidized.

In a preferred embodiment the reaction according to the invention is performed with
X is bromo.

In another preferred embodiment the reaction according to the invention is performed with
Br₂ as halogenating agent in step 1.

In another preferred embodiment the reaction according to the invention is performed with
Br₂ / H₂O₂, Br₂ /Oxone or Br₂ / NaOCl as halogenating agent in step 1.

In another preferred embodiment the reaction according to the invention is performed with NaBr or KBr/ H₂O₂, NaBr or KBr/ Oxone, NaBr or KBr/ NaOCl as halogenating agent in step 1.

In another preferred embodiment the reaction according to the invention is performed with HBr/H₂O₂, HBr /Oxone or HBr/ NaOCl as halogenating agent in step 1.

In another preferred embodiment the reaction according to the invention is performed with HCl, Cu₂O or CuO, NaNO₂ and 2-PrOH as reagent in step 2.

In another preferred embodiment the reaction according to the invention is performed with CuCl or CuCl₂, NaNO₂ and 2-PrOH as reagent in step 2.

In another preferred embodiment the reaction according to the invention is performed with CuSO₄, NaNO₂ and 2-PrOH as reagent in step 2.

The present invention has the advantage, that the halogenation is conducted with high atom efficiency and minimum waste production. It also leads to increased yield for the deamination step and in addition higher catalyst flexibility and process robustness.

### Elucidation of the processes and intermediates

The reaction scheme shows the two-step synthesis according to the invention.

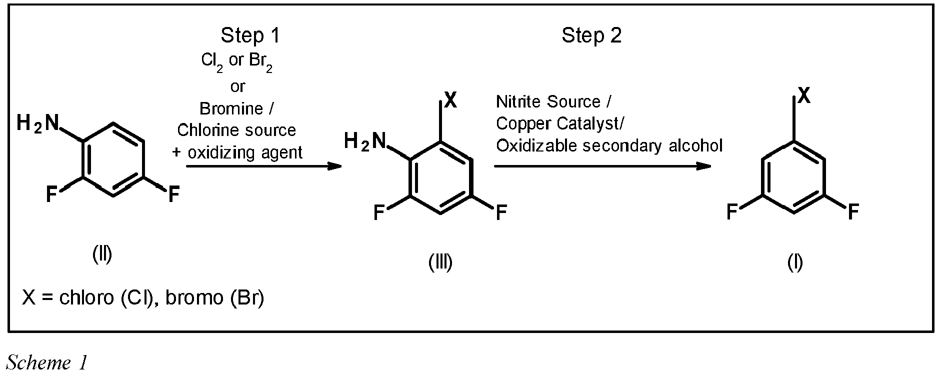

Process for preparing 1-halo-3,5-difluorobenzene of the formula **(I)**, characterized in that compounds of the general formula (**II**) react in a first step in the presence a bromine or chlorine source and an oxidizing agent to compounds of the general formula **(III)** and is in a second step is further converted to compounds of the formula **(I)** in the presence of a nitrite source, a copper catalyst and a secondary alcohol that can be oxidized.

### Step 1:

In the process according to the invention 0.45 to 1.5, preferably 0.55 to 1.2 equivalents bromine or chlorine source is used.

### For reactions with H₂O₂/HBr

In the process according to the invention 0.9 to 1.5, preferably 0.99 to 1.2 equivalents H₂O₂ is used.

In the process according to the invention 0.9 to 1.5, preferably 0.99 to 1.2 equivalents HBr is used.

### For reactions with H₂O₂/NaBr or KBr

In the process according to the invention 0.9 to 1.5, preferably 0.99 to 1.2 equivalents H₂O₂ is used.

In the process according to the invention 0.9 to 1.5, preferably 0.99 to 1.2 equivalents NaBr or KBr is used.

In a preferred embodiment, stochiometric amounts of acid, for example H₂SO₄, HCl, HBr, H₃PO₄ or boric acid are added.

These stoichiometric quantities can be applied analogously for the other oxidizing agents as mentioned above.For *reactions with Br₂*

In the process according to the invention 0.90 to 1.5, preferably 0.99 to 1.2 equivalents Br₂ is used.

### For reactions with H₂O₂/Br₂

In the process according to the invention 0.40 to 0.6 , preferably 0.45 to 0.55 equivalents Br₂ is used.

In the process according to the invention 0.45 to 0.65, preferably 0.50 to 0.60 equivalents H₂O₂ is used.

### Temperature range for the halogenation

The halogenation is usually performed in a temperature range from -10°C to 60°C, preferably -5°C to 50°C, particularly preferably 0 to 40°C.

### Solvent for the halogenation

The halogenation is furthermore performed in the presence of a solvent or diluent, preferred solvents being, water, toluene, chlorobenzene, ethyl acetate, isopropyl acetate, methyl-tert-butyl ether, cylopentylmethylether, methyl-tetrahydrofuran, tert-amylmethylether, Dichloromethane, Dichloroethane or mixtures of the above mentioned solvents.

### Step 2:

In the process according to the invention 0.01 to 0.5 equivalents. , preferably 0.05 to 0.1 equivalents copper reagent is used.

In the process according to the invention 0.95 to 1.2 equivalents , preferably 0.99 to 1.1 equivalents of the nitrite source are used.

In the process according to the invention sodium nitrite (NaNO₂) or potassium nitrite (KNO₂) is used as nitrite source, preferably sodium nitrite is used.

In the process according to the invention as oxidizable alcohol 2-propanol or 2-butanol is used, preferably 2-propanol is used.

### Temperature range for the reductive deamination

The reductive deamination is usually performed in a temperature range from -10°C to 40°C, preferably - 0°C to 50°C, particularly preferably 0 to 30°C.

### Solvent for the reductive deamination

The reductive deamination is furthermore performed in the presence of a solvent or dilutent, preferred solvents are water, aqueous solutions of HBr or HCl or mixtures of those.

### Examples

The present invention is elucidated in more detail by the examples that follow, without restriction of the invention thereto.

### Measurement methods

The products were characterized by ¹H spectroscopy and/or HPLC and/or GC-MS (Liquid Chromatography Mass Spectrometry) and / or GC

The NMR spectra were determined using a ECZL 400S NMR (JEOL 40 MHz NMR).

The GC chromatograms were measured on Shimadzu instrument, connected to a mass detector.

### Step 1

### Example 1.1: Preparation of 5-Bromo-2,4-difluoroaniline

To 200 g 2,4-difluoroaniline in a two-liter reactor is added 300 g of a 47% HBr solution over a time period of 15 minutes at a temperature of 28 to 30°C. Furthermore 188 g of MTBE (Methyl-tert-butylether) and 240 g of water is added at that temperature. Stirring is continued for further 15 minutes. Then 125 g of a 48 % H₂O₂ solution is added at 30 to 32 °C over 2 hours. It is stirred for 4 hours. Then the reaction mixture is cooled to 15°C, 296 g of MTBE is added. It is stirred for 30 minutes. Then stirring is stopped and the separation of phases is awaited for 30 minutes. The aqueous phase is extracted with MTBE. The combined organic layers are evaporated to yield the 320 g of 5-Bromo-2,4-difluoroaniline which is dissolved in 231 g isopropyl alcohol and directly used for the next step without further purification.

In accordance with example 1.1 (Step 1), the following examples have been prepared:

| Example | Bromine Source | Oxidant | Additive | Solvent | Yield |
|---|---|---|---|---|---|
| 1.2^{a)} | NaBr | H₂O₂ | H₂SO₄ | /H₂O | 90% |
| 1.3 | NaBr | H₂O₂ | Boric acid + H₂SO₄ | H₂O | 97% |
| 1.4^{a)} | NaBr | H₂O₂ | H₂SO₄ | MTBE/H₂O | 89% |
| 1.5^{b)} | NaBr | H₂O₂ | H₂SO₄ | MTBE/H₂O | 94% |
| 1.6 | HBr | H₂O₂ | - | H₂O | 92% |
| 1.7 | HBr | H₂O₂ | - | MTBE/H₂O | 99% |

| | | | | | |
|---|---|---|---|---|---|
| ^{a)} H₂SO₄ added after H₂O₂ addition. ^{b)} Addition of H₂SO₄ followed by H₂O₂. | | | | | |

### Step 2

### Example 2.1:Preparation of 1-Bromo-3,5-difluorobenzene

A flask is charged with 936 g 30% HCl, followed by 7.6 g of CuCl, 222 g of water and then 320 g of 5-Bromo-2,4-difluoroaniline which is dissolved in 231 g isopropyl alcohol (step 1). Stirring for 1 hour at 25°C and then cooling to 0°C. Then 318 g of a 40% NaNO₂ solution (127.2 g NaNO₂ and 190.8 g water) at 0 to 2°C is added over 4 hours. Further stirring for 30 minutes at 0°C to 2 °C. Then raise temperature slowly to 25°C and stir for 3 to 4 hours. Then 1000 g water is added. Stirring for 15 minutes at 25°C. Then 888 g MTBE is added and stirring for 30 minutes at 25°C. Wait for settling for 30 minutes. Then separation of phases. Aqueous phase is extracted with MTBE. Organic phases are combined. The solvent is distilled off. The product is distilled out of the crude to yield a colourless liquid. (256 g , GC purity > 98% 86% over two steps).

GC-MS: 113.05 (192/194, M⁺)

¹H-NMR (CDCl₃): 7.10 - 7.05 (m, 2H), 6.78 (tt, 1H) ppm.

In accordance with example 1.2 (Step 2), the following examples have been prepared:

| Example | Nitrite Source | Catalyst (eq) | Alcohol | Solvent | Yield over 2 Steps |
|---|---|---|---|---|---|
| Preparation of 1-Bromo-3,5-difluorobenzene according to US patent - 5,977,412 | | | | | 14% |
| Preparation of 1-Bromo-3,5-difluorobenzene according to CN105949067. | | | | | 19-20% |
| 2.2 | NaNO₂ | CuCl₂ (0.05) | iPrOH | HCl | 86% |
| 2.3 | NaNO₂ | Cu₂O (0.05) | iPrOH | HCl | 83% |
| 2.4 | NaNO₂ | CuSO4 (0.05) | iPrOH | HCl | 86% |
| 2.5 | NaNO₂ | CuCl (0.025) | iPrOH | HCl | 87% |
| 2.6^{a)} | NaNO₂ | CuCl (0.05) | iPrOH | HCl | 68% |
| 2.7^{b)} | NaNO₂ | CuCl (0.05) | iPrOH | HCl | 72% |
| 2.8^{c)} | NaNO₂ | CuCl (0.05) | iPrOH | HCl | 87% |

| | | | | | |
|---|---|---|---|---|---|
| ^{a)} Direct use of reaction mixture from example 1.5 without intermediate work-up. ^{b)} Extraction of product from step 1 with HCl required for step 2. | | | | | |

## Claims

1. Process for preparing 1-halo-3,5-difluorobenzene of the formula **(I)** 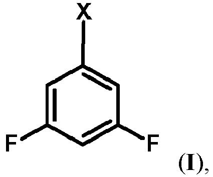 **characterized in that** compound of the formula **(II)** 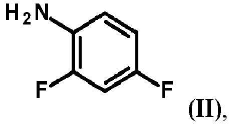 reacts in a first step in the presence of bromine or chlorine or a combination of a bromine or chlorine source and an oxidizing agent
to compounds of the formula **(III)** 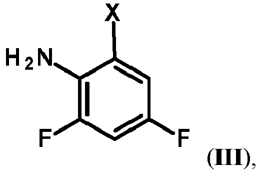 wherein
X is bromo or chloro,
and is, in a second step, further converted to compounds of the formula **(I)** in the presence of a nitrite source, a copper catalyst and a secondary alcohol that can be oxidized.

2. Process according to Claim 1,
wherein X is bromo.

3. Process according to Claim 1, wherein the reaction according to the invention is performed with Br₂ as halogenating agent in step 1.

4. Process according to Claim 1, wherein the reaction according to the invention is performed with Br₂ / H₂O₂, Br₂ /Oxone or Br₂ / NaOCl as halogenating agent in step 1.

5. Process according to Claim 1, wherein the reaction according to the invention is performed with NaBr or KBr/ H₂O₂,NaBr or KBr/ Oxone, NaBr or KBr/ NaOCl as halogenating agent in step 1.

6. Process according to Claim 1, wherein the reaction according to the invention is performed with HBr/H₂O₂, HBr /Oxone or HBr/ NaOCl as halogenating agent in step 1.

7. Process according to Claim 1, wherein the reaction according to the invention is performed with HCl, Cu₂O or CuO, NaNO₂ and 2-PrOH as reagent in step 2.

8. Process according to Claim 1, wherein the reaction according to the invention is performed with CuCl or CuCl₂, NaNO₂ and 2-PrOH as reagent in step 2.

9. Process according to Claim 1, wherein the reaction according to the invention is performed with CuSO₄, NaNO₂ and 2-PrOH as reagent in step 2.
